Europäisches Patentamt

European Patent Office

Office européen des brevets

(11) Publication number: **0 232 142 B1**

(12) **EUROPEAN PATENT SPECIFICATION**

(45) Date of publication of patent specification: **11.11.92** (51) Int. Cl.5: **G01L 9/06**, G01L 19/14

(21) Application number: **87300823.9**

(22) Date of filing: **30.01.87**

(54) **Pressure transducer.**

(30) Priority: **04.02.86 US 826056**

(43) Date of publication of application:
**12.08.87 Bulletin 87/33**

(45) Publication of the grant of the patent:
**11.11.92 Bulletin 92/46**

(84) Designated Contracting States:
**BE CH DE ES FR GB IT LI NL SE**

(56) References cited:
**EP-A- 0 124 308**

**MACHINE DESIGN, vol. 56, August 1984, no. 19, Cleveland, Ohio, US, page 60; "Throw-away chip monitors blood pressure"**

**WESCON PROCEEDINGS SAN FRANSISCO, 19th-22nd November 1985, 29 (1985) November, New York, US, pager 27/4, pages 1-5; R. FRANK: "An update on the integration of silicon pressure sensors"**

**ELECTRONIC DESIGN, vol. 33, April 1985, no. 9, Hasbrouck Heights, New Jersey, US, pages 193-196, 198; R. FRANK: " One-element transducer brings uniform traits to IC pressure sensors"**

(73) Proprietor: **Deseret Medical, Inc.**
**9450 South State Street**
**Sandy Utah 84070(US)**

(72) Inventor: **Hanlon, Stephen Paul**
**10045 So. Ridge Gate Circle**
**Sandy Utah 84092(US)**
Inventor: **Kerby, Walter Lynn**
**1623 Casper Road**
**Sandy Utah 84092(US)**
Inventor: **Purdy, Edmund Robert**
**1102 East Raymond Road**
**Fruitheights Utah 84037(US)**
Inventor: **Strom, James**
**2216 Atkins Avenue**
**Salt Lake City Utah 84109(US)**

(74) Representative: **Jones, Michael Raymond et al**
**HASELTINE LAKE & CO. Hazlitt House 28**
**Southampton Buildings Chancery Lane**
**London WC2A 1AT(GB)**

## Description

This invention relates to a pressure transducer assembly, which may be suitable for use in a medical context. Electrocardiograms (ECGs) which monitor the electrical activity of the heart are known, but the presence of an ECG signal does not indicate how effectively the blood is pumped through the arteries. Direct vessel blood pressure measurements are more accurate and reliable indicators of the state of the circulatory system. It is also known that accurate and continuous measurement of arterial and venous blood pressures can be obtained by placement of a catheter, attached to a pressure transducer, directly into the circulatory system, the vascular pressure being transmitted through fluid (usually saline) which is placed in the catheter.

Arterial blood pressure pulsates between peak (systolic) and minimum (diastolic) pressures. For a healthy young adult, these pressures are typically about 120 and 80 mm Hg, respectively, usually expressed as systolic over diastolic, or 120/80. Such arterial blood pressure monitoring may be necessary when an accurate assessment of the state of the circulatory system is required (e.g. during surgical procedures or following myocardial infarction). Similarly, continuous arterial pressure monitoring is used to estimate vital organ perfusion, to manage and monitor therapy, and to follow a patient's response to treatment during a hypertensive crisis.

The arterial pressure waveform often provides useful information on the pumping action of the heart and aortic valve closure. Observing the left radial arterial waveform is a standard procedure when positioning intra-aortic balloon pumps to assist circulation, i.e. the pressure waveform gives early knowledge of mechanical problems with the monitoring line, such as a clotted catheter.

Central venous pressure (CVP) is usually measured in the superior vena cava, and typically ranges from 3 to 9 mm Hg above atmospheric pressure. A partial vacuum (below atmospheric pressure) of a few mm Hg can be created intermittently by respiratory efforts. CVP is monitored when fluids are being lost (e.g. in burn victims or patients undergoing major surgery) or gained (e.g. in patients receiving large infusions of blood or other fluids) because of its relationship to vascular fluid volume. Low CVP is a factor which can indicate hypovolemia (inadequate blood volume) due to haemorrhage or dehydration, and can precede or accompany shock. Excessively high CVP may accompany hypervolemia (excessive blood volume), pulmonary edema, or other forms of heart failure.

Two types of blood pressure can be measured in the pulmonary circulatory system, either the pulmonary artery pressure (PAP) or the pulmonary artery wedge pressure (PAWP). PAWP was difficult to measure before the introduction of the flow-directed, balloon-tipped catheter. This device has made catheter placement quicker, more effective, and safer, without the need for routine fluoroscopy. PAWP is closely related to the left atrial pressure and left ventricular end diastolic pressure. Increased PAWP has proved to be one of the earliest indications of left heart failure. Mean PAWP is normally about 5-12 mm Hg, but it may rise significantly with heart failure. PAWP measurements are also used to determine optimal cardiac output, and serve as guidelines for administering fluids and diuretics to control intravascular volume and drugs that increase the contractility of the heart, thereby decreasing the work of the heart.

Pressure transducers are most commonly used for blood pressure monitoring, but they can also be used to measure intracranial, intrauterine, urinary bladder, oesophageal and other selected pressures. To measure intracranial pressure (ICP), a small fluid-filled catheter may be introduced into the subdural or epidural space, or into the ventricles of the brain, for ICP transmission to an external transducer. Intracranial pressure is slightly pulsatile, and can vary from a normal of 10 mm Hg to 80 mm Hg. Excessive arterial $CO_2$ concentration (tension), high arterial blood pressure, trauma to the head, and certain drugs can elevate ICP. High ICP levels that persist for more than a few minutes can cause cerebral dysfunction or brain death. Monitoring ICP permits immediate recognition of potentially dangerous ICP levels, and shows the effects of drugs or other means of reducing ICP to normal levels.

Uterine contractions during labour and delivery are sometimes monitored by direct intrauterine pressure (IUP) measurements. A long, fluid-filled catheter is introduced into the uterus and connected to a pressure transducer. IUP monitoring serves to measure the effect of oxytocin during the induction of labour. When labour has begun, changes in the IUP during uterine contractions may be recorded along with the foetal heart rate to facilitate the early detection of foetal distress.

Pressure measurements within the urinary bladder during urination are sometimes helpful in evaluating certain diseases of the bladder and urethra. Similarly, pressure measurements are sometimes used in diagnosing diseases involving the oesophagus. With one or more balloons positioned in the oesophagus, pressures within the balloon(s) are measured as the patient swallows. Oesophageal pressure measurements may also be made to determine the effects of respiration on PAWP.

Known physiological pressure transducers have been reusable devices. The electrical elements used in reusable transducers are strain-sensitive resistive wires, or semiconductor elements, that are bonded to a diaphragm. A capacitor or inductor can be used, instead of the resistive elements, to measure the displacement of the diaphragm. The resistive elements are connected to other resistors to form a Wheatstone bridge circuit, such that when an excitation voltage is applied across the bridge, the voltage output of the bridge is proportional to the displacement of the diaphragm.

Disposable transducers would have several advantages over traditional reusable units. By using disposables, hospitals could stock many units, and thus always have enough transducers to meet the demands of the monitors available. The time and economic losses associated with repairing damaged, and replacing lost, reusable transducers, especially miniature ones (accidental loss of transducers in dirty linen is not uncommon) would be eliminated. A patient could then be moved from one hospital area to another without the need to change transducers and/or monitoring kits, or to keep track of the location of the transducer. Additionally, the user would always have a new device that had not been subjected to the abuses (e.g. dropping, or impacting diaphragm) that a reusable could be exposed to during prior use and reprocessing (cleaning and sterilization).

An electrical risk related to direct blood pressure monitoring exists when pressures are measured in or near the heart. A saline-filled blood pressure catheter provides a direct conductive pathway between monitoring equipment and the heart, it being possible that a low electrical current could reach the heart causing microshock and ventricular fibrillation. Current could enter the catheter via the transducer if there were inadequate electrical isolation between energized elements of the transducer (or parts of the case that can be touched by personnel attending the patient) and the saline. Direct blood pressure measurements can often be made on patients undergoing electrosurgery; it is possible that some current from the ESU may pass through the transducer. Ideally, the transducer should not be damaged by this current, nor subject to excessive drift due to heating effects.

A pressure monitoring system arrives in the medical professions's hands air-filled. Before use it must be purged and filled with fluid (saline). During this filling process, air bubbles may be trapped, which are compressible and may compromise the dynamic response of the monitoring system, which results in non-faithful waveform reproduction. A flow path that contains steps, discontinuities, or that is non-uniform may serve to capture small or large air bubbles. These bubbles are not only difficult and time consuming to remove but, if not completely removed, will affect the waveform fidelity as described above.

The dynamic response of a pressure monitoring system is normally limited more by length of the catheter extension tubing, compliance, and air bubbles in the fluid path than by the monitor or the transducer. Determining the dynamic response of the transducer alone will not demonstrate how well a given physiological waveform will be reproduced. The dynamic response of the system and the indwelling catheter will only determine the error in reproducing the pressure waveform. The response should be sufficient to reproduce the value of the systolic and diastolic pressures to within 5% of the original waveform with no significant distortion.

In EP-A-0124308 there is disclosed a transducer assembly having a housing defining a chamber which is divided into a first chamber and a second chamber by an insulated body attached on both its upper and lower sides to the housing. The first chamber is adapted to receive fluid flowing through the inlet and outlet ports of the transducer assembly, and contains the pressure transducer which is separated from the fluid flowing in the first chamber by an insulating medium across which fluid pressure can be determined.

According to the present invention there is provided a pressure transducer assembly for monitoring fluid pressure comprising:

a hollow body (10) having an open chamber and a passage defining a flow path between two ports, there also being an opening (11d) in the flow path which extends to the chamber;

support means (14, 15, 17) locatable within the chamber and having first (15) and second sides (17), the support means having a cavity which leads to an opening in the first side (15), the first side (15) being securable relative to the flow path opening (11d), with the opening in the first side (15) communicating with the opening in the flow path;

a pressure transducer means (13) situated within the cavity and secured to an internal surface (17) of the support means, for converting fluid pressure into first electrical signals;

a fluid pressure responsive medium (16) which is electrically non-conductive and occupies the cavity between the flow path and the transducer (13);

electrical conducting means (20) connected to the pressure transducer means (13) and extending beyond the support means (14, 15, 17), at least part of the extending region being formed as electrical connectors; and

integrated circuit means (21) connectable to the electrical connectors (20) and also connectable to an output cable, for modifying the first electrical signals from the transducer (13) to produce second electrical signals which are capable of being read, via the output cable, on a measuring instrument,

the arrangement being such that, in use of the assembly in assembled form and with the passage filled with fluid, the fluid pressure, and any change therein, is transmitted through the fluid pressure responsive medium (16) to the transducer means (13) which converts the fluid pressure into first electrical signals, the first electrical signals being converted by the integrated circuit means (21) into second electrical signals which are capable of being read, via the output cable, on a measuring instrument,

characterized in that the passage defining the flow path is a uniformly dimensioned passage,

and in that the support means (14, 15, 17) is so mounted in the hollow body (10) that direct impacts on the outside of the assembly will not be directly absorbed by the pressure transducer (3).

Micromachining and silicone production techniques allow the transducer employed in the assembly of the present invention to be made from an etched pressure diaphragm with diffused piezoresistors for measuring displacement, and hence the transducer means can be made quite small. Since the diaphragm and sensing elements are integral, there is a reduction of offset pressure signals due to thermoelastic strain between sensing elements and the diaphragm. Zero-drift and inaccurate measurements are minimized. The silicon diaphragm is a nearly perfect elastic material (i.e., it exhibits very little "memory" or hysteresis). The high sensitivity of the sensor diaphragm permits miniaturization reducing the displacement volume and improving frequency response.

The integrated circuit means is contained on a thick film resistor network that may be laser trimmed to remove offset voltages and to set precisely its sensitivity to the same level as that of most reusable transducers (5 microvolt output per volt excitation per mm Hg pressure). The laser trimming also sets the temperature compensation. Thick film technology is employed to minimize the circuit size of the integrated circuit means, which may be an impedance matching portion, and which is preferably disposed within the housing beneath the transducer diaphragm.

For most disposable pressure transducer assemblies, the resistance of their bridge elements must be high enough to overcome self-heating effects, which can cause erroneous measurements. High resistance can result in an output impedance sufficient to cause an error when used with some blood pressure monitors. The combination of a high-output-impedance transducer and a low-input-impedance monitor may load the transducer, resulting in improper transfer of the pressure signal from the transducer to the monitor and an incorrect pressure display. Each pressure transducer assembly includes its own impedance circuitry or buffer.

Where an impedance load exists, active electronics have been used to buffer the impedance of the pressure transducer assembly so that it is compatible with a monitor. It has been of concern to users that the pressure transducer assembly be provided with the proper cable, including buffering circuits, for the type of monitor to which it is connected. Active electronic cables intended to compensate for the high output impedance of the transducer element are designed to remain connected to their respective monitors. The excitation voltage normally supplied by the monitor to the transducer is used to power the active electronic circuitry in these buffering circuits.

In addition to the care with which the pressure transducer assembly and monitor are matched to one another, it is also important that the pressure transducer assembly be physically rugged enough to withstand handling and use and still provide accurate, reliable and repeatable pressure measurements. As explained, a thin silicon chip is etched to provide the transducer means. The positioning and mounting of this delicate chip is critical to the performance of the transducer means during pressure measurement no matter how severe normal handling becomes. Consequently, the way in which the chip is mounted and is exposed for and associated with the saline column of fluid pressure measurement is critical to the accuracy and performance of the device.

In addition to the concern of shock hazard, there is also concern for over pressuring the transducer means thereby shifting its calibration or destroying its dielectric integrity. Some transducers require an additional port for flushing air bubbles from the system, and for zeroing the transducer means to atmospheric pressure, prior to monitoring. The preferred structure is such that it is resistant to overpressure to a greater degree than any available pressure transducer assembly and is configured to minimize the difficulty of debubbling.

The preferred pressure transducer assembly is geometrically configured such that it may be easily used, in a disposable fashion, in combination with monitoring equipment and other equipment designed for both disposable and reusable transducers. Preferably, the pressure transducer assembly of the present invention has a cylindrical tube which fits in-line with the administration set, flush device and the like, by means of luer lock connections designed to be compatible with the particular apparatus required for access to the monitored pressure fluctuations of the patient.

Preferably, the opening in the first side is cylindrical and normal to the path of flow; and the first side is provided with upstanding arcuate portions which conform to portions of the passage, in the region of the opening in the flow path, and which, in combination with the fluid pressure responsive medium, are capable of forming a smooth tubular flow path of uniform cross-section between the two ports.

Preferably, the first side is sealed within a recess in the hollow body, and the first side is also sealed to the opening in the flow path in order to allow fluid communication between the flow path and the transducer means.

Preferably, the support means is formed of an electrically insulating material.

Preferably the fluid pressure responsive medium is electrically non-conductive.

Overlying the transducer element is the fluid pressure responsive medium which is conveniently a potting gel which has a high dielectric constant and is designed to seal the transducer from direct contact with the saline fluid of the system whereby the corrosive effects and conductive effects of the saline fluid are insulated from the diaphragm of the transducer. Such a gelatinous dielectric acts as an insulating or isolating sealant to permit only pressure fluctuations to reach the transducer diaphragm. The gel is preferably situated to form a part of the wall of the cylindrical flow path and, therefore, does not obstruct the ease of debubbling. The fluid pressure responsive medium, which is preferably an insulating, isolating, and supporting gel is specifically formulated for ease of potting, electrical isolation, good sealing qualities, stability in manufacturing and anti-photo sensitivity. With regard to the latter, the gel may include a colourant which prevents light from having any effect on the transducer.

Preferably there is an opening in the second side of the support means, and preferably the pressure transducer means is exposed to the opening in the second side of the support means.

Preferably the opening in the second side is coaxial with the opening in the first side, and both openings are centrally located in the support means.

Preferably the pressure transducer means is resiliently secured to the second side by the fluid pressure responsive medium.

Preferably the second side is provided with a recess which is capable of supporting and protecting the transducer means which may be resiliently secured therein, and the second side has a peripheral flange in the region of the opening in the second side, for providing a mounting place for the pressure transducer means.

Preferably the integrated circuit means includes signal modifying means capable of appropriately setting impedance from the transducer means to a measuring instrument for analyzing and recording the information transduced.

Preferably a hollow cover is provided, which is capable of cooperating with the hollow body to seal the chamber to form an enclosure, the cover preferably having a flange insertable within the chamber of the body.

Preferably, the output cable is cooperatively engaged by both the body and the cover to relieve strain in the region where the output cable connects with the integrated circuit means.

Preferably, the integrated circuit means is carried by the electrical conducting means within the enclosure formed by the body and the cover, and the inside surface of the cover does not in any way interfere with, or engage, any portion of the integrated circuit means or the electrical conducting means, such that the integrated circuit means is resiliently suspended from the support means via the electrical conducting means, without being subjected to any stresses from the body or the cover.

Because of the geometry of the cylindrical flow path and its orientation relative to the administration set and the catheter, the system is easily debubbled. Over all, the structure is rugged and has the right relationship of housing strength for support to prevent problems of over pressure. Syringes used to flush bubbles from within transducer domes and pressure tubing, to draw blood samples from monitoring lines, and to introduce drugs can be misused to dislodge clots from arterial catheters.

The transducer diaphragm is adequately supported such that pressure fluctuations are the only stress to which it is capable of responding. The full surface of the diaphragm is exposed to the pressure fluctuations but is not subjected directly to impact received by the body and cover within which the transducer is carried. That is to say, the diaphragm is shock mounted within the unit so that direct loadings on the outside will not be directly absorbed by the transducer diaphragm. Similarly, the laser trimmed compensation circuitry is preferably fully suspended to eliminate the effects of impact upon that delicate ceramic micro circuit board. The electrical output from the diaphragm is connected to the laser trimmed compensation circuit supported just beneath the diaphragm thereby minimizing losses incurred and elongated connections between the diaphragm and the compensation circuitry. The circuit can be located in that part of the enclosure defined by the cover. Signals from the diaphragm in response to pressure fluctuations are amplified, filtered, and suitably modified by the circuitry integral with the cover and body that carry the diaphragm. Preferably the output signal is impedance matched and is sent through an ambient air pressure compensating cable to a specifically designed matched connection from the computer and readout apparatus. That connection is unique in that it caries both electrical signals and air pressure whereby venting of the underside of the diaphragm is remote from the diaphragm and matching circuitry housing.

For a better understanding of the present invention and to show how the same may be carried into effect, reference will now be made, by way of example, to the accompanying drawings, in which:

Figure 1 is an exploded perspective view of a preferred embodiment of a pressure transducer assembly according to the present invention, showing all of its relevant components; and

Figure 2 is a cross-sectional view, essentially along the line 2-2 of Figure 1, showing the relative relationship of the transducer means to the flow path, and the mounting of the same, as well as the laser trimmed ceramic micro circuit board.

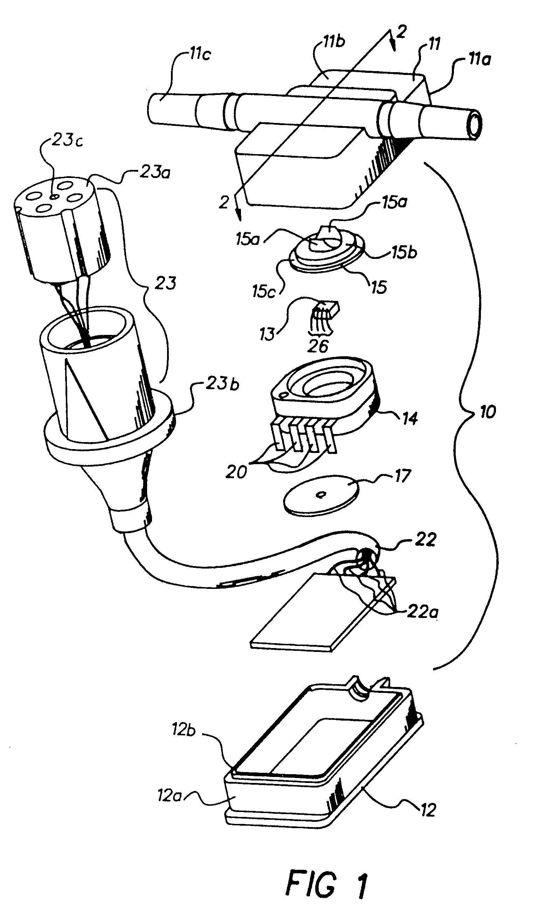

In Figure 1, there is shown a housing 10, which forms an enclosure, composed of a body 11 and a cover 12. The body 11 is a hollow rectangular case, which defines an open chamber, with a wall 11a and a top 11b having a transverse tubular member 11c extending transversely across the top 11b of the body 11. Tubular member 11c encloses the flow path which has an opening of circular cross-section, for matching with the connections to the administration set and the patient, and forms a uniform flow path. The cover 12 has a wall 12a which is configured to cooperatively engage with wall 11a on cover 11 by means of an upstanding flange 12b that extends thereabout around the top edge of the wall 12a. The flange 12b is positioned to fit just inside the wall 11a to form a lip type seal therebetween. Cover 12 is also hollow and case like, forming an open chamber, and when cooperating with body 11 forms the housing 10 which is an enclosure for other components of the pressure transducer assembly.

In a manner well known, the tubular member 11c can be connected to an administration set on one side and a catheter on the other, whereby pressure pulses from the patient are hydraulically transmitted via a saline solution; the pressure in the saline solution can be transmitted via the pressure responsive medium to the transducer 13 in the housing 10. In this embodiment the transducer 13 is a thin semiconductor film resistive strain gauge element which is mounted within a support means 14, 15, 17 in a manner which will expose the transducer to pressure fluctuations within the flow path through the tubular member 11c.

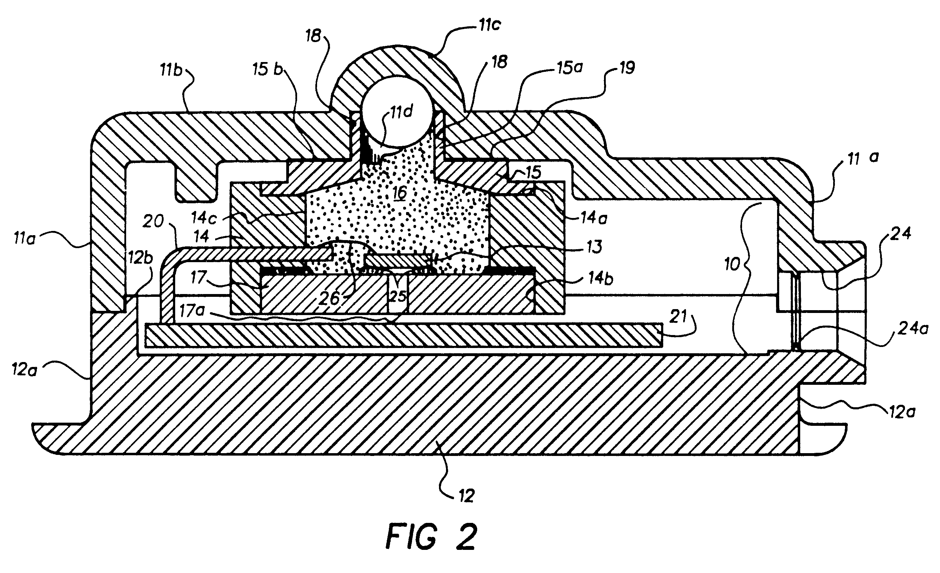

There is a first side 15 of the support means which is connected to the tubular member 11c. The relative relationship of the aforesaid components is best seen in Figure 2, which also shows the interrelationship between the upstanding flange 12b and the wall 11a. These components are bonded together at that juncture in the final assembly. In addition, the relative positions of the body 11 and the first side 15 are clearly disclosed, and the relationship of the flow path through the tubular member 11c to the transducer 13. In that regard, there is disposed between the flow path and the transducer 13 a fluid pressure-responsive medium 16 in the form of a silicone gel.

The fluid pressure responsive medium 16 is located within the cavity of the support means 14, 15, 17. A sealing disk 17 forms the second side of the support means 14, 15, 17. There is an opening 11d extending from the flow path of the tubular member 11c downwardly into the hollow central portion of the housing 10. Opening 11d is generally circular in cross-section and slightly larger in one direction than the diameter of the flow path whereby recesses 18 exist and accept upstanding arcuate portions 15a on the first side 15 of the support means, thus forming a straight cylindrical hollow opening from the tangent point of the diameter of the flow path downwardly into the housing 10. Figure 2 clearly shows the manner in which the upstanding arcuate portions 15a cooperate with the recesses 18 of the opening 11d and form a part of the flow path.

On the first side of the support means 15, and radiating outwardly from the upstanding arcuate portions 15a, is a top shoulder 15b, which is adapted to be a mounting area against the inside of the top 11b of the body 11. An adhesive layer 19 is positioned between the shoulder 15b and the inside of the top 11b such that the first side 15 of the support means is adhesively secured to the body 11. The first side 15 of the support means also includes a radiating rim 15c, the outer periphery of which is essentially circular. Rim 15c conjugates within an upwardly open circular recess 14a, of a support housing 14, in mating engagement to centre the housing 14 axially with respect to the cylindrical opening 11d of the body 11. Therefore, components 11, 15, and 14 are all similarly aligned along a common axis (not shown). Support housing 14 also has a bottom circular recess 14b fashioned to accept therein the second side 17 of the support means, shaped as a sealing disk. Disposed between the recess 14a and the recess 14b is an inwardly extending web 14c composed of a thickened wall section in the centre of the support housing 14. Consequently, the sealing disk 17 and a central opening 17a therein are coaxial with the support housing 14, the first side 15 of the support means, and the body 11. While coaxial alignment is not critical, it does provide a datum about which the hollow recess to be filled with fluid pressure responsive medium 16 is defined, thus easing the filling of the hollow with the medium 16.

In the illustrated embodiment, the central opening 17a has a specific purpose in that the pressure transducer 13 is mounted thereabove such that ambient air pressure can reach the bottom side of the pressure transducer 13. Consequently, pressure fluctuations transmitted through the pressure responsive medium 16 act upon one side of the transducer 13, and are measured against ambient air pressure on the other side thereof. The pressure transducer 13 is disposed immediately above the central opening 17a. Sealing disk 17 is metallic, preferably aluminium, and is adhesively bonded and slightly press fitted into the recess 14b of support housing 14. The adhesive used in the preferred embodiment is one known as RTV. While the final assembled position of the relative components can be seen in Figure 2, there is a preferred method used to assemble them. Before explaining that particular procedure, various other components of the assembly ought to be described.

As can be seen in Figures 1 and 2, there are four electrical connectors, which in this embodiment are buss bars 20, extending through one side of the wall of the inwardly extending web 14c of the support housing 14 and outwardly and downwardly therefrom. These buss bars 20 are moulded into the support housing 14 and are made of highly conductive metal such as copper or phosphor bronze. Suspended from the downwardly extending buss bars 20 is an integrated circuit means, which in this embodiment is a ceramic circuit board 21. Circuit board 21 in the preferred embodiment includes the following circuitry; impedance matching, temperature compensating, and trimming networks. The buss bars 20 offer support and electrical connection to the circuit board 21, which as shown in figure 1 is connected to an output cable 22 by means of wires 22a. The cable 22 extends to an outlet connector 23 composed of a female socket 23a and a manipulative housing 23b. The wiring connections with respect to the cable 22 and its wires 22a are not shown in Figure 2; however, those skilled in the art will no doubt appreciate that the cable 22 is fitted through an opening 24 in the side of the housing 10 whereby, in a well-known manner, strain is relieved in the region where the output cable 22 connects with the integrated circuit means 21. In this particular circumstance, there is a rib 24a which circumscribes the inside of the opening 24 and provides a contact point against the outside sheath of cable 22. In order to further secure the combination, a mounting adhesive is applied between the juncture of the cable 22 and the opening 24, particularly in the area of the rib 24a.

As shown in Figure 2, the transducer means 13 is resiliently bonded to the sealing disk 17 by means of the application of a resilient adhesive 25. The preferred technique for assembly of the pressure transducer starts with the transducer means 13 and disk 17. The transducer means 13 is adhesively bonded, above the opening 25, to the sealing disk 17 and the combination is adhesively bonded into the support housing 14, more particularly the disk 17 being seated in recess 14b. Thereafter the transducer means 13 is connected to the buss bars 20 by connecting wires 26 shown in Figures 1 and 2. A small amount of fluid pressure responsive medium 16 is then introduced into the cavity formed within the web 14c of the support housing 14 to just fill the area defined by the first side 15 of the support means. Then the assembly of the first side 15 to the support housing 14 takes place. At this stage, the fluid pressure responsive medium 16 is not shaped to form the concave circular bottom for the flow path through tubular member 11c. That procedure is carried out when the first side 15 of the support means is ready to be assembled with the recesses 18 of the body 11.

Thus, it can be seen that the application of the pressure responsive medium 16 is a two-step process, the latter step being to form the concave circular bottom of the flow path through the tubular member 11c. At that point, the support means 14, 15, 17, is pressed up against the inside of body 11 with solvent 19 (methylene chloride), to form a bonded interface 19, being applied to the surface of shoulder 15b and the inside of top 11b to form a tight bond therebetween. As shown in Figure 1, the upstanding arcuate portions 15a are aligned to form the uniform circular flow path through the tubular member 11c of the body 11. the output cable 22 and its connecting wires 22a are then connected to the integrated circuit means 21 carried on the buss bars 20 such that it is suspended as shown in Figure 2. The cable 22 is laid into the opening 24 and against the "strain relief" rib 24a of the body 11. The cover 12 is then bonded, by means of an adhesive applied along the juncture between the upstanding flange 12b and the inside wall 11a of the body 11, to the body 11 once some more adhesive is placed on the juncture between the cable 22 and the opening 24 and the package is tightly conjugated to form the housing 10 as a sealed package. In the cable 22 is an air passage 23c shown in Figure 1 which equalizes the air pressure inside the housing 10 with that outside the housing. The socket 23a connects with a male socket (not shown) and extends to a computer which is designed to receive, analyze and record the signals from the transducer.

Those skilled in the art will appreciate that a suspended shock mounting has been formed for not only the pressure transducer 13, but also the integrated circuit means 21. In particular, the transducer 13 is resiliently bonded, by means of adhesive 25, to the sealing disk 17 which is also resiliently bonded to the inside of recess 14b of the support housing 14. The latter is carried by the first side 15 of the support means which is adhesively secured to the inside of the body 11. Any reasonable impact imposed upon the cover 12 or the body 11 has no direct effect on either the pressure transducer or the ceramic microcircuit 21 because of the adhesive bonding and the mounting by means of buss bars 20. It should be appreciated that the concept sought to be protected in the claims which follow also covers the shock mounting as well as the uniform circular flow path, the former giving the product its ruggedness and the latter making the system easy to debubble. Therefore, alterations in the basic geometry or configurations, materials used or techniques for fabrication are within the scope of the claims.

Throughout this specification the abbreviation ESU has been used for Electrosurgery.

## Claims

1. A pressure transducer assembly for monitoring fluid pressure comprising:

a hollow body having an open chamber and a passage defining a flow path between two ports, there also being an opening in the flow path which extends to the chamber;

support means locatable within the chamber and having first and second sides, the support means having a cavity which leads to an opening in the first side, the first side being securable relative to the flow path opening, with the opening in the first side communicating with the opening in the flow path;

a pressure transducer means situated within the cavity and secured to an internal surface of the support means, for converting fluid pressure into first electrical signals;

a fluid pressure responsive medium which is electrically non-conductive and occupies the cavity between the flow path and the transducer;

electrical conducting means connected to the pressure transducer means and extending beyond the support means, at least part of the extending region being formed as electrical connectors; and

integrated circuit means connectable to the electrical connectors and also connectable to an output cable, for modifying the first electrical signals from the transducer to produce second electrical signals which are capable of being read, via the output cable, on a measuring instrument,

the arrangement being such that, in use of the assembly in assembled form and with the passage filled with fluid, the fluid pressure, and any change therein, is transmitted through the fluid pressure responsive medium to the transducer means which converts the fluid pressure into first electrical signals, the first electrical signals being converted by the integrated circuit means into second electrical signals which are capable of being read, via the output cable, on a measuring instrument,

characterized in that the passage defining the flow path is a uniformly dimensioned passage,

and in that the support means is so mounted in the hollow body that direct impacts on the outside of the assembly will not be directly absorbed by the pressure transducer.

2. A pressure transducer assembly according to claim 1, wherein the support means forms a suspended shock mounting for the pressure transducer.

3. A pressure transducer assembly according to claim 1 or 2, wherein the opening in the flow path is cylindrical and normal to the flow path between the ports.

4. A pressure transducer assembly according to claim 1, 2 or 3, wherein the first side of the support means is shaped so as to form, in combination with the fluid pressure responsive medium, part of the passage of uniform cross-section defining the flow path between the two ports.

5. A pressure transducer assembly according to claim 4, wherein the first side of the support means is shaped by the provision of upstanding arcuate portions which conform to portions of the passage in the region of the opening in the flow path.

6. A pressure transducer assembly according to any preceding claim, wherein the first side is sealed within a recess in the hollow body, and the first side is also sealed to the opening in the flow path in order to allow fluid communication between the flow path and the transducer means.

7. A pressure transducer assembly according to any preceding claim, wherein the support means is formed of an electrically insulating material.

8. A pressure transducer assembly according to any preceding claim, wherein there is an opening in the second side of the support means, and wherein the pressure transducer means is exposed to the opening in the second side of the support means.

9. A pressure transducer assembly according to claim 8, wherein the opening in the second side is coaxial with the opening in the first side, and both openings are centrally located in the support means.

10. A pressure transducer assembly according to claim 8 or 9, wherein the pressure transducer means is resiliently secured to the second side by the fluid pressure responsive medium.

11. A pressure transducer assembly according to claim 10, wherein the second side is provided with a recess which is capable of supporting and protecting the transducer means which may be resiliently secured therein, and the second side has a peripheral flange in the region of the opening in the second side, for providing a mounting place for the pressure transducer means.

12. A pressure transducer assembly according to any preceding claim, wherein the integrated circuit means includes signal modifying means capable of appropriately setting impedance from the transducer means to a measuring instrument for analyzing and recording the information transduced.

13. A pressure transducer assembly according to any preceding claim, wherein a hollow cover is provided, which is capable of cooperating with the hollow body to seal the chamber to form an enclosure, the cover preferably having a flange insertable within the chamber of the body.

14. A pressure transducer assembly according to claim 13, wherein the output cable is cooperatively engaged by both the body and the cover to relieve strain in the region where the output cable connects with the integrated circuit means.

15. A pressure transducer assembly according to claim 13 or 14, wherein the integrated circuit means is carried by the electrical conducting means within the enclosure formed by the body and the cover, and the inside surface of the cover does not in any way interfere with, or engage, any portion of the integrated circuit means or the electrical conducting means, such that the integrated circuit means is resiliently suspended from the support means via the electrical conducting means, without being subjected to any stresses from the body or the cover.

**Patentansprüche**

1. Druckmeßwandlervorrichtung zur Überwachung von Fluiddruck, mit

   einem Hohlkörper mit einer offenen Kammer und einem Kanal, der einen Fließweg zwischen zwei Ports definiert, wobei ferner in dem Fließweg eine Öffnung vorgesehen ist, die sich zu der Kammer erstreckt;

   einer Haltevorrichtung, die innerhalb der Kammer angeordnet werden kann und eine erste und eine zweite Seite aufweist, wobei die Haltevorrichtung einen Hohlraum aufweist, der zu einer Öffnung in der ersten Seite führt, wobei die erste Seite relativ zu der Fließwegöffnung festlegbar ist, derart, daß die Öffnung in der ersten Seite mit der Öffnung in dem Fließweg verbunden ist;

   einer in dem Hohlraum angeordneten und an einer Innenfläche der Haltevorrichtung befestigten Druckmeßwandlereinrichtung zum Umsetzen von Fluiddruck in erste elektrische Signale;

   einem auf Fluiddruck reagierenden Medium, das elektrisch nichtleitend ist und den Hohlraum zwischen dem Fließweg und dem Druckmeßwandler einnimmt;

   einer elektrisch leitenden Einrichtung, die mit der Druckmeßwandlereinrichtung verbunden ist und über die Haltevorrichtung hinausragt, wobei mindestens ein Teil des hinausragenden Bereiches in Form von elektrischen Konnektoren ausgebildet ist; und

   einer mit den elektrischen Konnektoren und ferner mit einem Ausgangskabel verbindbaren integrierten Schaltung zum Modifizieren der ersten elektrischen Signale von dem Wandler zur Erzeugung von zweiten elektrischen Signalen, die über das Ausgangskabel an einem Meßinstrument ablesbar sind,

   wobei die Anordnung derart vorgesehen ist, daß bei Benutzung der Vorrichtung im montierten Zustand und bei mit Fluid gefülltem Kanal der Fluiddruck sowie dessen Änderungen durch das auf Fluiddruck reagierende Medium an die Meßwandlereinrichtung übermittelt werden, die den Fluiddruck in erste elektrische Signale umsetzt, wobei die ersten elektrischen Signale mittels der integrierten Schaltung in zweite elektrische Signale umgesetzt werden, die über das Ausgangskabel an einem Meßinstrument ablesbar sind,

dadurch gekennzeichnet, daß der den Fließweg defi-nierende Kanal ein gleichförmig dimensionierter Kanal ist,

und daß die Haltevorrichtung derart in dem Hohlkörper montiert ist, daß direkte Stöße auf die Außenseite der Vorrichtung nicht direkt von dem Druckmeßwandler absorbiert werden.

2. Druckmeßwandlervorrichtung nach Anspruch 1, bei der die Haltevorrichtung eine hängende stoßdämpfende Halterung für den Druckmeßwandler bildet.

3. Druckmeßwandlervorrichtung nach Anspruch 1 oder 2, bei der die Öffnung in dem Fließweg zylindrisch ist und senkrecht zu dem Fließweg zwischen den Ports verläuft.

4. Druckmeßwandlervorrichtung nach Anspruch 1, 2 oder 3, bei der die erste Seite der Haltevorrichtung so geformt ist, daß sie in Kombination mit dem auf Fluiddruck reagierenden Medium einen Teil des gleichförmigen Querschnitt aufweisenden Kanals bildet, der den Fließweg zwischen den beiden Ports definiert.

5. Druckmeßwandlervorrichtung nach Anspruch 4, bei der die erste Seite der Haltevorrichtung durch dazu vorgesehene aufrechte bogenförmige Teile ausgebildet ist, die im Bereich der in dem Fließweg befindlichen Öffnung Teilen des Kanals konform sind.

6. Druckmeßwandlervorrichtung nach einem der vorhergehenden Ansprüche, bei der die erste Seite innerhalb einer Vertiefung in dem Hohlkörper abgedichtet ist, und die erste Seite ferner zu der in dem Fließweg befindlichen Öffnung abgedichtet ist, um Fluidverbindung zwischen dem Fließweg und der Meßwandlervorrichtung zuzulassen.

7. Druckmeßwandlervorrichtung nach einem der vorhergehenden Ansprüche, bei der die Haltevorrichtung aus elektrisch isolierendem Material gebildet ist.

8. Druckmeßwandlervorrichtung nach einem der vorhergehenden Ansprüche, bei der eine Öffnung in der zweiten Seite der Haltevorrichtung ausgebildet ist, und bei der die Druckmeßwandlereinrichtung der Öffnung in der zweiten Seite der Haltevorrichtung ausgesetzt ist.

9. Druckmeßwandlervorrichtung nach Anspruch 8, bei der die Öffnung in der zweiten Seite koaxial mit der Öffnung in der ersten Seite ist und beide Öffnungen mittig in der Haltevorrichtung angeordnet sind.

10. Druckmeßwandlervorrichtung nach Anspruch 8 oder 9, bei der die Druckmeßwandlereinrichtung durch das auf Fluiddruck reagierende Medium elastisch an der zweiten Seite angebracht ist.

11. Druckmeßwandlervorrichtung nach Anspruch 10, bei der die zweite Seite eine Vertiefung aufweist, die imstande ist zur Stützung und zum Schutz der Meßwandlereinrichtung, welche elastisch darin gehalten sein kann, und die zweite Seite zur Schaffung eines Anbringungsortes für die Druckmeßwandlereinrichtung einen Umfangsflansch im Bereich der in der zweiten Seite befindlichen Öffnung aufweist.

12. Druckmeßwandlervorrichtung nach einem der vorhergehenden Ansprüche, bei der die integrierte Schaltung eine Signalmodifizierungseinrichtung aufweist, die zur korrekten Einstellung der Impedanz von der Meßwandlereinrichtung zu einem Meßinstrument zur Analyse und Aufzeichnung der umgesetzten Information imstande ist.

13. Druckmeßwandlervorrichtung nach einem der vorhergehenden Ansprüche, bei der eine hohle Abdeckung vorgesehen ist, die imstande ist, derart mit dem Hohlkörper zusammenzuwirken, daß sie die Kammer unter Bildung eines Einschlusses abdichtet, wobei die Abdeckung vorzugsweise einen Flansch aufweist, der in die Kammer des Körpers einführbar ist.

14. Druckmeßwandlervorrichtung nach Anspruch 13, bei der sowohl der Körper als auch die Abdeckung an dem Ausgangskabel zusammenwirkend angreifen, zur Zugentlastung in dem Bereich, in dem das Ausgangskabel mit der integrierten Schaltung verbunden ist.

15. Druckmeßwandlervorrichtung nach Anspruch 13 oder 14, bei der die integrierte Schaltung von der elektrisch leitenden Einrichtung innerhalb der durch den Körper und die Abdeckung gebildeten Einfassung gehalten ist und die Innenfläche der Abdeckung in keiner Weise mit irgendeinem Teil der integrierten Schaltung oder der elektrisch leitenden Einrichtung in Konflikt gerät oder an diesen angreift, derart, daß die integrierte Schaltung über die elektrisch leitende Einrichtung elastisch von der Halteeinrichtung herabhängt, ohne durch den Körper oder die Abdeckung Spannungen ausgesetzt zu sein.

**Revendications**

1. Un transducteur de pression pour le contrôle de la pression de fluide comprenant :

un corps creux ayant une chambre ouverte et un passage définissant une voie d'écoulement entre deux orifices, avec une ouverture dans la voie d'écoulement qui va dans la chambre ;

un moyen de support pouvant être placé dans la chambre et ayant un premier et un second côtés, le moyen de support ayant une cavité qui conduit à une ouverture dans le premier côté, le premier côté pouvant être fixé par rapport à l'ouverture de la voie d'écoulement, l'ouverture du premier côté communiquant avec l'ouverture dans la voie d'écoulement ;

un transducteur de pression situé dans la cavité et fixé à une surface interne du moyen de support pour transformer la pression du fluide en premiers signaux électriques ;

un moyen sensible à la pression du fluide qui est électriquement non-conducteur et occupe la cavité entre la voie d'écoulement et le transducteur ;

un moyen électriquement conducteur relié au transducteur de pression et disposé au-delà du moyen de support, au moins une partie de cette région étant constituée de connecteurs électriques ; et

un système de circuits intégrés pouvant être relié aux connecteurs électriques et également pouvant être relié à un câble de sortie, pour modifier les premiers signaux électriques provenant du transducteur de façon à produire de seconds signaux électriques qui peuvent être lus, par le câble de sortie, sur un appareil de mesure,

la disposition étant telle que, lors de l'utilisation de l'ensemble monté et en remplissant le passage de fluide, la pression du fluide et tout changement de celle-ci soit transmise par le milieu sensible à la pression du fluide au transducteur qui transforme la pression du fluide en premiers signaux électriques, les premiers signaux électriques étant transformés par le système de circuits intégrés en seconds signaux électriques qui peuvent être lus, par le câble de sortie, sur un appareil de mesure.

caractérisé en ce que le passage définissant la voie d'écoulement est un passage de dimension uniforme,

et en ce que le moyen de support est monté dans le corps creux de telle sorte que les chocs directs sur l'extérieur de l'ensemble ne soient pas directement absorbés par le transducteur de pression.

2. Un ensemble transducteur de pression conforme à la revendication 1, caractérisé en ce que le moyen de support forme un amortisseur de choc suspendu pour le transducteur de pression.

3. Un ensemble transducteur de pression conforme à la revendication 1 ou 2, caractérisé en ce que l'ouverture dans la voie d'écoulement est cylindrique et normale à la voie d'écoulement entre les orifices.

4. Un ensemble transducteur de pression conforme à la revendication 1, 2 ou 3, caractérisé en ce que le premier côté du moyen de support a une forme telle qu'il contitue en liaison avec le moyen sensible à la pression du fluide, une partie du passage de section transversale uniforme définissant la voie d'écoulement entre les deux orifices.

5. Un ensemble transducteur de pression conforme à la revendication 4 caractérisé en ce que le premier côté du moyen de support est formé par la disposition de parties droites en forme d'arc qui épousent la forme des parties du passage dans la région d'ouverture dans la voie d'écoulement.

6. Un ensemble transducteur de pression conforme à l'une des revendications précédentes, caractérisé en ce que le premier côté est fermé hermétiquement dans un évidement du corps creux, et le premier côté est également fermé hermétiquement à l'ouverture dans la voie d'écoulement afin de permettre la communication du fluide entre la voie d'écoulement et le transducteur.

7. Un ensemble transducteur de pression conforme à l'une des revendications précédentes, caractérisé en ce que le moyen de support est formé d'une matière électriquement isolante.

8. Un ensemble transducteur de pression conforme à l'une des précédentes revendications, caractérisé en ce qu'il y a une ouverture dans le second côté du moyen de support, et en ce que le transducteur de pression est exposé à l'ouverture dans le second côté du moyen de support.

9. Un ensemble transducteur de pression conforme à la revendication 8, caractérisé en ce que l'ouverture dans le second côté est co-axiale à l'ouverture dans le premier côté, et les deux ouvertures sont situées au centre du moyen de support.

10. Un ensemble transducteur de pression conforme à la revendication 8 ou 9, caractérisé en ce que le transducteur de pression est fixé de façon élastique au second côté par le milieu sensible à la pression du fluide.

11. Un ensemble transducteur de pression conforme à la revendication 10, caractérisé en ce que le second côté est muni d'un évidement qui peut supporter et protéger le transducteur qui peut y être monté élastiquement, et le second côté a une bride périphérique dans la région de l'ouverture dans le second côté, pour constituer un lieu de montage du transducteur de pression.

12. Un ensemble transducteur de pression conforme à l'une des précédentes revendications, caractérisé en ce que le système de circuits intégrés comprend un moyen de modification des signaux pouvant régler convenablement l'impédance du transducteur à un appareil de mesure pour analyser et enregistrer les informations transmises.

13. Un ensemble transducteur de pression conforme à l'une des revendications précédentes, caractérisé en ce qu'un couvercle creux est prévu, pouvant coopérer avec le corps creux pour fermer hermétiquement la chambre de façon à former une enceinte, le couvercle ayant de préférence une bride pouvant être insérée dans la chambre du corps.

14. Un ensemble transducteur de pression conforme à la revendication 13, caractérisé en ce que le câble de sortie est engagé en coopération avec le corps et le couvercle pour soulager les contraintes dans la région où le câble de sortie se raccorde au système de circuits intégrés.

15. Un ensemble transducteur de pression conforme à la revendication 13 ou 14, caractérisé en ce que le système de circuits intégrés est porté par le moyen électriquement conducteur dans l'enceinte formée par le corps et le couvercle, et la surface intérieure du couvercle ne gêne en aucune façon, ni n'entre en contact avec une partie du système de circuits intégrés ou du moyen électriquement conducteur, de telle sorte que le système de circuits intégrés est suspendu de façon élastique au moyen de support par le moyen électriquement conducteur, sans être soumis à des contraintes provenant du corps ou du couvercle.

FIG 1

FIG 2

EP 0 232 142 B1